# FASCICULE DE BREVET EUROPEEN

(11) **EP 2 912 005 B1**
(45) Date de publication et mention de la délivrance du brevet: **15.08.2018**
(21) Numéro de dépôt: 13805427.5
(22) Date de dépôt: 17.10.2013
(51) Int. Cl.: C07C 45/38, C07C 45/75, C07C 47/22, C07C 47/06, C07C 47/04

(54) **PROCEDE DE SYNTHESE DIRECTE D'ALDEHYDES INSATURES A PARTIR DE MELANGES D'ALCOOLS**
VERFAHREN ZUR DIREKTEN SYNTHESE VON UNGESÄTTIGTEN ALDEHYDEN AUS ALKOHOLGEMISCHEN
METHOD FOR DIRECTLY SYNTHESISING UNSATURATED ALDEHYDES FROM ALCOHOL MIXTURES

(30) Priorité: 29.10.2012 FR 1260298
(43) Date de publication de la demande: 02.09.2015
(73) Titulaire: Arkema France, 92700 Colombes (FR)
(72) Inventeur: DUBOIS, Jean-Luc, F-69390 Millery (FR); CAPRON, Mickaël, F-59830 Bachy (FR); DUMEIGNIL, Franck, F-59273 Fretin (FR)
(74) Mandataire: Bonnel, Claudine
(86) Numéro de dépôt international: PCT/FR2013/052477
(87) Numéro de publication internationale: WO 2014/068213

(56) Documents cités:
- WO-A1-2005/040392
- WO-A1-2011/093763
- C. E. BLOM ET AL: "Molecular structure of s-cis- and s-trans-acrolein determined by microwave spectroscopy", JOURNAL OF THE AMERICAN CHEMICAL SOCIETY, vol. 106, no. 24, 1 novembre 1984 (1984-11-01), pages 7427-7431, XP055071969, ISSN: 0002-7863, DOI: 10.1021/ja00336a022
- WANG F-I ET AL: "CATALYTIC SYNTHESIS OF ISOBUTYRALDEHYDE FROM METHANOL AND ETHANOL OVER TITANIUM OXIDE-SUPPORTED VANADIUM OXIDE CATALYSTS", JOURNAL OF THE CHEMICAL SOCIETY, CHEMICAL COMMUNICATIONS, CHEMICAL SOCIETY. LETCHWORTH, GB, no. 24, 15 décembre 1991 (1991-12-15), XP000242116, ISSN: 0022-4936, DOI: 10.1039/C39910001760
- SCHULZ H ET AL: "SYNTHESE UND UMWANDLUNGSPRODUKTE DES ACROLEINS", ANGEWANDTE CHEMIE, WILEY - V C H VERLAG GMBH & CO. KGAA, DE, vol. 62, no. 5, 7 mars 1950 (1950-03-07), pages 105-118, XP000653944, ISSN: 0044-8249, DOI: 10.1002/ANGE.19500620502

## Description

La présente invention a pour objet un procédé de synthèse directe d'acroléine ou de méthacroléine à partir d'un mélange de méthanol et d'éthanol ou de propanol.

L'acroléine est le nom usuel donné à l'aldéhyde insaturé de formule CH₂=CH-CHO appelé propénal, et la méthacroléine celui donné au composé CH₂=C(CH₃)-CHO, ou 2-méthylpropénal.

L'acroléine est connue depuis fort longtemps et est utilisée comme biocide à large spectre ; elle est également un intermédiaire pour la synthèse de divers produits tels que la D,L-Méthionine (supplément pour nourriture animale), de l'acide acrylique, des produits pharmaceutiques, des fongicides, des parfums, de la pyridine, des picolines, du glutaraldéhyde, etc.

La méthacroléine est également connue depuis fort longtemps et est essentiellement utilisée notamment pour la synthèse de l'acide méthacrylique et du méthacrylate de méthyle ainsi que pour la synthèse de polymères et de résines synthétiques.

Les grands procédés industriels connus de fabrication de l'acroléine sont aujourd'hui celui développé par la société DEGUSSA au cours des années 1940 utilisant comme matières premières l'acétaldéhyde et le formol, et celui mis en place quelque 20 ans plus tard par diverses sociétés consistant à oxyder le propylène. Plus récemment, un procédé de production d'acroléine par déshydratation du glycérol a été développé par la demanderesse.

Le procédé DEGUSSA, qui est illustré par le brevet UK 513,772 déposé le 31 mars 1938 sous priorité du 1^{er} avril 1937 et par l'article intitulé «Synthèse und Umwandlungsprodukte des Acroleins» dans Angewandte Chemie 62. Jahrgang. Nr.5. Seite 105-132, paru le 7 mars 1950 conduit en phase gazeuse par catalyse hétérogène est fondé sur la réaction d'aldolisation suivante :

CH₃-CHO + CH₂O → CH₂=CH-CHO + H₂O

L'article Journal of the American Chemical Society, 1984, 106, pp.7427-7431*,* décrit des conditions de synthèse d'un isotope ¹³C de l'acroléine par réaction d'aldolisation du formaldéhyde avec de l'acétaldéhyde résultant de l'oxydation d'éthanol (¹³C).

Le deuxième procédé qui est illustré dans les Techniques de l'Ingénieur, traité Génie des procédés J6 100-pages 1 à 4 a supplanté industriellement le précédent procédé et est fondé sur la réaction d'oxydation du propylène suivante :

CH₂=CH-CH₃ + O₂ → CH₂=CH-CHO + H₂O

ledit procédé utilisant des catalyseurs oxydes mixtes à base de Molybdène - Bismuth et Fer décrits dans « Procédés de Pétrochimie », A. Chauvel, G. Lefebvre et L. Castex, Tome 2, Editions Technip, 2° Edition de 1986 pages 213-219 ; un tel procédé est également décrit dans la fiche catalyse 34 de la Division Catalyse de la Société Française de Chimie.

Plus récemment, des travaux importants ont été réalisés par la demanderesse (voir la demande de brevet WO 2011/083225) pour faire évoluer un autre procédé de synthèse à partir de glycérol soumis à une déshydratation selon la réaction suivante :

CH₂OH-CHOH-CH₂OH → CH₂=CH-CHO + 2H₂O

Ce type de procédé présente l'avantage de pouvoir travailler avec une matière première naturelle renouvelable (non fossile) et donc de répondre aux engagements de la plupart des pays industrialisés visant à réduire les émissions de gaz à effet de serre avec ses effets environnementaux, mais aussi de fournir une solution de production plus durable.

La synthèse de la méthacroléine utilise généralement comme matières premières l'isobutane, le tertiobutanol ou l'isobutène qui sont transformés en méthacroléine par oxydation. Il existe d'autres procédés de synthèse tels que l'oxydéshydrogénation de l'isobutaraldéhyde ou l'hydroformylation du méthylacétylène ou du propadiène. Outre dans certains cas leur coût, l'origine fossile de ces matières premières est un désavantage majeur.

Comme on l'a vu ces divers procédés présentent des inconvénients.

Les procédés industriels fondés sur l'oxydation d'oléfines consomment des matières premières fossiles, extraites du pétrole notamment, dont le coût d'accès devient de plus en plus élevé. Par ailleurs, leur disponibilité est liée à des installations de raffinage ou de pétrochimie de grandes capacités ce qui impose en pratique une implantation à proximité des grands sites pétrochimiques ce qui, implique donc des coûts de transport vers le consommateur final sans compter les risques d'émissions de produits polluants.

Les procédés mettant en oeuvre la réaction d'aldolisation nécessitent des investissements importants dans la mesure où, si l'on souhaite s'affranchir de l'achat des matières premières - formol et acétaldéhyde - qui ne se stockent ni ne se transportent facilement, par exemple, le formol peut être transporté uniquement sur un rayon de 400 km autour du site de production, et doit pour cela être stabilisé avec du méthanol en solution aqueuse - il est nécessaire de prévoir des unités spécifiques de synthèse de ces produits qui s'ajoutent au coût de l'unité d'aldolisation proprement dite. La synthèse de l'acétaldéhyde est généralement réalisée par la réaction de Waker d'oxydation de l'éthylène et celle du formol par oxydation (ou oxydéshydrogénation selon le système catalytique retenu) du méthanol.

Les procédés mettant en oeuvre la déshydratation du glycérol sont attrayants du fait de la nature renouvelable de la charge traitée. Cependant, ils présentent certains désavantages sur plusieurs plans. En effet, l'approvisionnement en glycérol dépend principalement des unités oléochimiques et/ou de biodiésel. Le glycérol n'est disponible qu'en quantités limitées ce qui restreint en pratique la taille possible des unités de production d'acroléine et donc leur rentabilité économique. Les qualités de glycérol les plus répandues sont dites de Glycérine brute, ce qui correspond le plus souvent à des solutions aqueuses de glycérol à 80 % poids contenant des sels (par exemple NaCl, KCl, Na₂SO₄, K₂SO₄...), du méthanol résiduel quand il a été obtenu par méthanolyse des huiles végétales, des Matières Organiques Non Glycérineuses (MONG), et toutes sortes d'impuretés extraites des plantes ou graisses animales lors des procédés de production. Ces impuretés sont des poisons pour les catalyseurs et conduisent à une désactivation réversible, par exemple par formation de coke, ou irréversible, par exemple par dépôt des sels. De la glycérine raffinée est aussi disponible commercialement, mais à des prix beaucoup plus élevés. Sur un plan technique, les catalyseurs de déshydratation connus jusqu'à maintenant se désactivent rapidement ce qui nécessite des régénérations régulières impliquant un surcoût en investissement (par exemple un doublement ou triplement des volumes catalytiques). Enfin, ce procédé «vert» présente un inconvénient du fait que la réaction de déshydratation, effectuée en phase gaz, globalement forte consommatrice d'énergie est dépendante du coût de cette énergie aujourd'hui fournie à partir de combustibles fossiles ayant aussi obligatoirement un impact environnemental.

La demanderesse a donc recherché un procédé palliant les inconvénients précédents c'est-à-dire utilisant des matières premières d'origine renouvelable (non fossiles) et limitant au maximum les investissements. C'est ainsi qu'elle a découvert qu'il était possible de synthétiser l'acroléine et la méthacroléine par voie directe en utilisant comme charge un mélange de méthanol et d'éthanol (acroléine) ou de méthanol et de propanol (méthacroléine) composés disponibles à partir de matières premières renouvelables.

Dans le document Journal of the Chemical Society, Chemical Communications, 1991, 1760-1761*,* un mélange de méthanol et d'éthanol est converti en présence d'un catalyseur V/TiO₂ en un mélange comprenant de l'acroléine, de l'acétaldéhyde et de l'isobutyraldéhyde. Il n'y a pas d'oxygène dans le milieu réactionnel et le procédé conduit majoritairement à la formation d'aldéhydes saturés tel que l'isobutyraldéhyde. L'exemple 32 dans la demande WO 2005/040392 décrit l'oxydation d'un mélange de méthanol et d'éthanol en présence d'un catalyseur à l'argent, conduisant à un mélange d'acétaldéhyde et de formaldéhyde, qui est ensuite converti en acroléine en présence d'alumine. Cet exemple utilise un mélange réactionnel dont la composition n'est pas précisée, et nécessite d'adapter la température de réaction à chacune des étapes.

La demanderesse a maintenant découvert un procédé simplifié de synthèse d'acroléine à partir d'un mélange de méthanol et d'éthanol, en ce sens qu'il est mis en oeuvre par voie directe, en présence d'un catalyseur solide d'oxydation sélective et éventuellement d'un catalyseur solide de condensation par aldolisation.

La demanderesse a également trouvé que ce procédé s'applique à la synthèse de méthacroléine à partir d'un mélange de méthanol et de propanol.

Au sens de l'invention, on entend par voie directe, un procédé conduit dans un seul ensemble réactionnel. Cet ensemble réactionnel comporte soit un réacteur unique, soit deux réacteurs successifs mais dans ce cas, le second réacteur est alimenté, de préférence directement, par l'effluent de sortie du premier réacteur.

L'invention a pour objet un procédé de synthèse directe d'aldéhydes insaturés de formule CH₂ =C(R)-CHO dans laquelle R représente H ou CH₃ à partir d'une charge contenant un mélange de méthanol et d'un second alcool de formule R-CH₂ -CH₂ OH, consistant à introduire dans un seul ensemble réactionnel comportant un réacteur unique, opéré en phase gazeuse à une température comprise entre 200 et 400 °C et sous une pression comprise entre 1 et 10 bars absolus, contenant un lit catalytique constitué d'un unique catalyseur d'oxydation sélective solide choisi parmi les catalyseurs à base de molybdène ou d'un mélange physique d'un catalyseur d'oxydation sélective solide à base de molybdène avec un catalyseur solide de condensation par aldolisation, une charge contenant le mélange les deux alcools avec de l'oxygène et un gaz diluant non réactif, de telle sorte que le mélange d'alcools et l'oxygène représentent chacun au maximum 10% du volume total du mélange réactionnel puis, à la sortie de l'ensemble réactionnel à recueillir l'effluent gazeux comprenant l'aldéhyde insaturé formé, en présence d'eau coproduite par la réaction. Le procédé de l'invention consiste, dans une première phase à synthétiser simultanément par oxydation des alcools au contact du catalyseur d'oxydation, les deux aldéhydes de formules HCHO et R-CH₂-CHO, puis, dans une deuxième phase à réaliser au contact du catalyseur de condensation, une réaction de condensation croisée de ces deux aldéhydes (aldolisation) conduisant à la formation d'un aldol de formule CH₂OH-C(R)-CHO, qui par déshydratation catalytique conduit à l'aldéhyde insaturé.

La demanderesse a découvert de façon surprenante que les deux phases, à savoir les deux réactions successives d'oxydation et d'aldolisation, peuvent être effectuées en présence d'un catalyseur unique, notamment en présence d'un catalyseur d'oxydation sélective à base de molybdène.

Ainsi, dans le procédé de l'invention, on utilise un catalyseur à base de molybdène d'oxydation sélective des alcools légers de la charge, et éventuellement un catalyseur de condensation (aldolisation) des aldéhydes formés lors de l'oxydation.

Les catalyseurs d'oxydation sélective des alcools légers sont bien connus depuis des décennies. On connaît deux principaux procédés d'oxydation des alcools légers i) le procédé dit à l'argent où l'oxydation ou plutôt la déshydrogénation est conduite à haute température, environ 600 à 700°C tel que décrit dans le Catalyst Handbook M.V. Twigg publié par Wolfe Publishing Ltd (1989), Chapter 10. Catalytic Oxidations. Methanol Oxydation. 10.3.2 pages 490-499 (1999), et ii) un procédé mettant en oeuvre un catalyseur solide d'oxydation de type oxyde mixte à base de molybdène décrit dans le même ouvrage aux pages 499-503, procédé conduit à une température nettement plus basse, entre 300 et 400°C, avec des catalyseurs analogues à ceux utilisés dans le procédé FORMOX.

Dans le procédé de l'invention, on utilise un catalyseur solide d'oxydation de type oxyde mixte à base de molybdène.

Les catalyseurs d'oxydation utilisables dans le procédé de l'invention comprennent le molybdène et au moins un élément choisi parmi P, Si, W, Ti, V, Nb, Ta, Cr, Mn, Fe, Co, Ni, Cu, Zn, Ga, In, Tl, Sb, Ag, As, Ge, B, Bi, La, Ba, Sb, Te, Ce, Pb, choisis dans le groupe constitué par les oxydes mixtes contenant le molybdène et les hétéropolyacides contenant le molybdène.

Ces catalyseurs peuvent être représentés par la formule générale suivante.

**A**ₐ **X**_{b} **Mo**_{c} **Z**_{d} **O**ₑ

dans laquelle
A est au moins un cation choisi parmi les éléments des Groupes 1 à 16 de la Classification Périodique des Eléments et les lanthanides, de préférence un cation d'un métal alcalin tel que Cs, Rb ou K,
X est P ou Si, et de préférence P
Z est au moins un élément choisi dans le groupe comprenant par W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce and Pb et de préférence Cu, Fe, Bi, Co, Ni, W, V, Cr, Sb, Mn, Ce,
**O** est l'oxygène
a, b, c et d sont des indices constitués de nombres entiers ou décimaux satisfaisant aux plages suivantes
   0 ≤ a ≤ 9 et de préférence 0 < a ≤ 9
   0 ≤ b ≤ 2 et de préférence 0,1 ≤ b ≤ 1,5
   0 < c ≤ 12 et de préférence 5 < c ≤ 12
   0 ≤ d ≤ 12 et de préférence 0 < d ≤ 4
   tels que a + b + d > 0
et e est un nombre déterminé par le degré total d'oxydation des éléments.

Le catalyseur utilisé lors de cette étape d'oxydation pourra être un catalyseur du type Fe-Mo-O dopé ou non utilisé généralement lors de l'oxydation du méthanol en formaldéhyde. Des exemples de catalyseurs molybdate de fer (Fe-Mo-O) sont décrits dans Catalysis Review, Vol 47 (2005), pp 125-174*.* Ces catalyseurs sont disponibles commercialement. Par exemple Mapco, fournit un catalyseur avec sa licence de technologie et Süd Chemie fournit plusieurs grades de ces catalyseurs sous la marque FAMAX® : FAMAX J5, FAMAX MS, FAMAX HS, FAMAX TH. Dans le cas de ce type de catalyseur Fe-Mo, les indices a et b de la formule générale ci-dessus seront de préférence de valeur 0 et l'indice d sera de valeur non nulle.

De préférence, notamment dans la variante du procédé utilisant un catalyseur unique, le catalyseur d'oxydation est un catalyseur de type molybdate de fer.

Les catalyseurs d'oxydation peuvent être sous forme massique et utilisés dans ce cas sans support.

Les catalyseurs peuvent aussi être déposés sur un support inactif dont la quantité représente de 30 à 90% et de préférence au moins 50 % du poids total du catalyseur.

Il est possible d'utiliser comme support tout matériau tel que la stéatite, la silice, l'alumine, la magnésie, l'oxyde de titane, la zircone, le carbure de silicium, les silicates, les terres de diatomées, les borates ou carbonates, des céramiques, à condition que les matériaux soient stables aux conditions opératoires auxquelles les catalyseurs sont soumis.

Le catalyseur massique ou le catalyseur supporté peuvent être sous forme de granulé ou de poudre et se présenter sous une forme quelconque telle que sphère, grain, cylindre creux, trilobe et quadrilobe, ainsi que sous la forme de cylindres, extrudés ou compressés éventuellement en utilisant un agent de pastillage. De préférence le catalyseur est sous la forme de cylindres creux ou de trilobes creux.

Dans le procédé de l'invention, on peut utiliser tout catalyseur solide connu dans la littérature pour réaliser la condensation de deux aldéhydes et notamment du formol avec un aldéhyde supérieur (acétaldéhyde ou propanaldéhyde), pour conduire à l'aldéhyde insaturé correspondant en deux étapes : réaction d'aldolisation du formaldéhyde sur l'aldéhyde supérieur avec formation d'un aldéhyde hydroxylé (aldol) puis réaction de déshydratation de cet aldol.

Les catalyseurs de condensation utilisés dans le procédé de l'invention appartiennent aux différentes catégories suivantes.
1) les «bases supportées », c'est-à-dire les hydroxydes alcalins LiOH, NaOH, KOH, CsOH déposés sur support silice ou alumine, les métaux alcalins et alcalinoterreux dispersés sur silice, alumine (Al₂O₃-NaOH-Na), magnésie (MgO-NaOH), charbon ou carbonate de potassium, les composés azotés, NR₃, NH₃, KNH₂ déposés sur alumine, LiCO₃ déposé sur silice, t-BuOK déposé sur xonotlite et plus généralement les solides de type métaux alcalins déposés sur alumine (tels que Na/Al₂O₃ ou KF/Al₂O₃), sur silice ou sur magnésie (tel que Li/MgO).
   Ce catalyseur solide sera constitué par exemple de silicate de sodium déposé sur silice ou sur aluminosilicate présentant de préférence un rapport atomique Si/Al supérieur à 10 et comportant le cas échéant un promoteur métallique ou encore du césium déposé sur une silice greffée ou dopée par un composé du zirconium (Cs-Zr/SiO₂).
2) les «oxydes métalliques» BaO, BeO, SrO, CaO, Al₂O₃, Y₂O₃, La₂O₃, CeO₂, ThO₂, SnO₂, K₂O, Na₂O, MgO, ZnO, TiO₂, ZrO₂ sous forme oxyde ou carbonate éventuellement dopés avec un métal alcalin, les oxydes ou oxycarbonates de terres rares éventuellement dopés par des alcalins.
3) Les «sels métalliques» des composés ci-dessus, c'est-à-dire les carbonates, hydroxycarbonates, hydrogénocarbonates, sels d'ammonium, etc.
4) Les « oxydes mixtes » tels que SiO₂-MgO, SiO₂-CaO, SiO₂-SrO, SiO₂-BaO, SiO₂-SnO, SiO₂-ZnO, SiO₂-Al₂O₃, SiO₂-ThO₂, SiO₂-TiO₂, SiO₂-ZrO₂, SiO₂-MoO₃, SiO₂-WO₃, Al₂O₃-MgO, Al₂O₃-ThO₂, Al₂O₃-TiO₂, Al₂O₃-ZrO₂, Al₂O₃-MoO₃, Al₂O₃-WO₃, ZrO₂-ZnO, ZrO₂-TiO₂, TiO₂-MgO, ZrO₂-SnO₂. A cette liste d'oxydes mixtes, on peut ajouter les oxydes de type argile comme les hydrotalcites, hydroxyapatites, chrysolite et sépiolite, éventuellement dopés par des alcalins, ainsi que par des métaux tels que le cuivre, le fer, le nickel, ou les oxydes de terres rares dopés par des alcalino-terreux tels que (SrO-La₂O₃). Relèvent également de cette catégorie, d'autres catalyseurs qui peuvent aussi convenir pour cette réaction tels que les catalyseurs oxydes mixtes de type phosphates mixtes de cobalt et aluminium, ou silice-alumine dopées par exemple avec des sels de sodium (Na), potassium (K), césium (Cs), cadmium (Cd), Mg, Ca, Sr, Mn, Zn, Mo, Nb, Pb et/ou Si. Ils pourront être aussi MgO-alumine, MgO-SiO₂, des terres rares, sous forme de phosphates, tungstates, molybdates, etc. Les oxynitrures de dérivés phosphorés tels que les oxynitrures mixtes de Vanadium-Aluminium, Phosphore-Zirconium, Phosphore-Aluminium, Vanadium-Aluminium-Phosphore, Gallium-Aluminium-Phosphore peuvent aussi convenir pour cette réaction.
5) Différentes « zéolithes » échangées avec des ions alcalins (Cs, K, Na, Li).
On peut citer à titre d'exemple de tels catalyseurs, les aluminosilicates cristallisés ou amorphes, les silicalites, les zéolithes synthétiques cristallines telles que la Faujasite, la Ferriérite, la ZSM-5, sous leur forme acide ou sous une forme soit partiellement, soit totalement neutralisée par des éléments des groupes 1 à 14, et de préférence des groupes 1 et 2 et par Zn et Tl. Les zéolithes utilisées peuvent avoir dans leur structure une partie ou la totalité des atomes d'aluminium remplacés par des atomes trivalents tels que B, Ga, Fe, Cr, V, As, Sb, Bi, Be, et peuvent avoir une partie ou la totalité des atomes de silicium remplacés par des atomes tétravalents tels que Ge, Ti, Zr, Hf.

Les catalyseurs solides de condensation utilisés dans le procédé selon l'invention présentent une surface spécifique élevée, généralement comprise entre 40 et 600 m²/g et de préférence entre 50 et 200 m²/g.

Les propriétés acide-base d'un matériau peuvent être liées à sa composition par exemple, sa structure cristalline ou à des défauts de surface tels la présence d'impuretés comme les métaux alcalins ou alcalino-terreux.

La présence de sites basiques et leurs quantités peuvent être déterminées par toute méthode connue, par exemple par adsorption d'un composé acide tel que CO₂ ou SO₂ et par des mesures micro-calorimétriques de l'énergie d'adsorption. La présence de sites acides et leurs quantités peuvent quant à elles être mesurées par adsorption d'un composé basique tel que l'ammoniac par exemple.

Les mesures de l'acidité et de la basicité du catalyseur sont réalisées par adsorption de SO₂ (basicité) et de NH₃ (acidité) et par les mesures micro-calorimétriques de l'énergie d'adsorption. Les conditions opératoires habituelles sont les suivantes.

Les essais sont menés à 150°C dans un calorimètre (C80 de Setaram) relié à un appareil volumétrique conventionnel équipé d'un manomètre capacitif Barocel pour les mesures de pression. Les échantillons sont prétraités dans une cellule en quartz par chauffage pendant une nuit sous vide à 300°C. Cette température est atteinte par augmentation de température au rythme de 1°C/min. Les chaleurs différentielles d'adsorption sont mesurées comme en fonction du taux de recouvrement par envoi répétitif de petites doses des gaz respectifs sur l'échantillon jusqu'à ce qu'une pression d'équilibre de 67 Pa environ soit atteinte. L'échantillon est alors dégazé pendant 30 min. à la même température et une seconde série d'adsorption analogue est réalisée jusqu'à ce qu'à une pression d'équilibre de 27 Pa environ soit atteinte. La différence entre les quantités adsorbées entre la première et la seconde adsorption (à 27 Pa) représente la quantité adsorbée irréversiblement des gaz respectifs qui fournit une estimation du nombre de sites forts respectivement acide ou base.

Dans certains cas, l'acidité ou la basicité du solide n'est révélée que si une faible adsorption d'eau ou d'alcool est préalablement réalisée sur le solide. Dans le cas d'une atmosphère parfaitement sèche, la surface probablement déshydratée est moins acide et/ou basique. L'adsorption préalable d'eau ou d'alcool peut être représentative des conditions opératoires de la réaction.

Le procédé de l'invention comporte deux phases successives : oxydation puis condensation par aldolisation qui peuvent être effectuées en présence d'un catalyseur unique d'oxydation sélective à base de molybdène. Ces deux phases sont conduites dans un seul réacteur, Le réacteur comporte, soit un seul lit catalytique fixe constitué d'un unique catalyseur d'oxydation à base de molybdène ou d'un mélange physique des deux catalyseurs d'oxydation à base de molybdène et de condensation, soit de deux lits catalytiques superposés le premier (en amont) étant chargé d'un catalyseur d'oxydation et le second d'un catalyseur de condensation.

Dans cet ensemble réactionnel, les réactions sont conduites en phase gazeuse à une température comprise entre 200 et 400 °C et de préférence entre 250 et 350°C, sous une pression comprise entre 1 et 10 bars absolus et de préférence entre 1 et 5 bars absolus.

Le mélange d'alcools, méthanol et éthanol, ou méthanol et propanol selon les cas, est tel que les ratios molaires méthanol/éthanol et méthanol/propanol sont compris entre 0,8 et 2, en général entre 1 et 2 et de préférence entre 1,1 et 1,5.

Dans le cas d'un excès de l'un des réactifs, celui-ci pourra être avantageusement recyclé dans le réacteur sous la forme initiale alcool, ou sous la forme transformée en aldéhyde.

Le débit d'introduction du mélange d'alcools dans l'ensemble réactionnel est tel que la teneur totale en alcools du milieu réactionnel est comprise entre 4 et 10% et de préférence entre 6 et 9%, exprimée en volume.

Le débit d'introduction de l'oxygène sera tel que la teneur en oxygène du milieu réactionnel ne sera pas supérieure à 10 %, exprimée en volume.

Le reste du milieu réactionnel est constitué d'un ou plusieurs gaz inertes qui représentent de 80 à 88 % en volume du milieu réactionnel. La présence de ces derniers est indispensable pour éviter que le mélange ne soit situé dans la zone d'explosion. Ces gaz inertes seront par exemple de la vapeur d'eau, du gaz carbonique, de l'azote ou un gaz rare tel que l'argon ou l'hélium. Néanmoins, sous réserve d'équiper le réacteur de disques de ruptures ou autres équipements de sécurité, il est possible de fonctionner avec une concentration en oxygène supérieure à 10% en volume.

Les réactions sont conduites avec une VVH (vitesse volumique horaire) généralement comprise entre 2 000 et 40 000h⁻¹ et de préférence entre 10 000 et 20 000h⁻¹. La VVH est calculée en faisant le ratio entre le débit de gaz total (en normaux-litres) divisé par le volume de catalyseur (masse volumique apparente prise à 1 g/ml). Dans cette méthode, on ne tient compte que de la matière active et non des solides inertes utilisés pour diluer le catalyseur.

Les exemples suivants illustrent la présente invention sans toutefois en limiter la portée.

### Exemple 1 : Synthèse d'un catalyseur de condensation.

Le catalyseur de type Cs/Zr/SiO₂ est préparé à partir d'un gel de silice sous forme de particules de 315 à 500 microns ayant une pureté de 99,9%, une surface spécifique de 320 m²/g et un volume poreux de 0,9 cm³/g avec un diamètre de pore médian de 9 nm.

La silice est imprégnée par une solution de butoxyde de zirconium dans le butanol , filtration, et séchage en évaporateur tournant, puis dans un four à 120 °C pendant 2 heures. L'imprégnation et le séchage ont été répétés encore 2 fois de manière à obtenir un dépôt de 0,02 % en poids (1,2 g de zirconium pour 100 moles de silice). Le césium est alors lui aussi imprégné à partir d'une solution aqueuse de carbonate de césium, suivi d'un séchage, pour donner une teneur en césium d'environ 4 % pds (calculé en poids de métal). Le catalyseur a alors été calciné à 450 °C sous air pendant 3 heures. La surface spécifique du catalyseur ainsi préparé est de 147 m²/g.

### Exemple 2 : Synthèse d'un catalyseur de condensation

Le catalyseur Na/SiO₂ est préparé par imprégnation d'un gel de silice à gros pores, additionné de 12 % poids de silicate de sodium. Après imprégnation le solide est séché dans une étuve à 100°C pendant 10 h. puis calciné dans un four à 500°C pendant 3 heures. La surface spécifique du catalyseur préparé est de 122 m²/g.

### Exemple 3 : Synthèse de l'acroléine en présence d'un mélange de catalyseurs

On introduit dans un réacteur de 1 cm de diamètre interne 200 mg d'un mélange de catalyseur d'oxydation (A), Mapco MS de type molybdate de fer (FeMo), et de catalyseur de condensation (B) de type Na/SiO₂ (dénommé Na dans le tableau 1) ou Cs/Zr/SiO₂ (dénommé Zr dans le tableau 1) préparés comme décrit ci-dessus.

Le mélange de catalyseurs est soumis à un broyage et après tamisage on sélectionne la fraction de la poudre dont la granulométrie est comprise entre 80 et 100 µm. Cette poudre est diluée par une quantité équivalente de carbure de silicium (SiC) de granulométrie 105 µm. Enfin, 500mg de SiC ont été ajoutés au-dessus du lit catalytique ainsi constitué pour favoriser l'homogénéité et l'écoulement du flux réactionnel et du chauffage.

Le débit d'injection de la charge gazeuse préchauffée à 120 °C, est de 50 ml/minute, soit 3000 ml/h. Le mélange d'alcool est injecté liquide dans un évaporateur dans lequel il rencontre les autres gaz.

Le mélange gazeux de la charge a selon les essais la composition suivante : 8 ou 10 % d'oxygène, mélange équimoléculaire de 4 ou 5 % d'éthanol et de 4 ou 5 % de méthanol et le complément (80 à 84 %) d'Hélium (contenant 1 % de Kr comme étalon interne).

Le mélange d'alcools est alimenté par une pompe, passe par un évaporateur avant de rejoindre le flux gazeux en amont du réacteur.

Le réacteur est placé dans un four chauffé électriquement. Dans les essais qui suivent la réaction est effectuée à une pression très légèrement supérieure à la pression atmosphérique et à une température variant de 250 à 400°C.

Les effluents sont analysés par chromatographie avec détecteur FID (à ionisation de flamme) et avec détection par spectrométrie de masse.

Les résultats obtenus sont donnés dans le tableau 1 ci-dessous.

Les rendements en produits sont calculés en tenant en compte du nombre de carbones dans les réactifs et produits.

Ainsi le rendement en acroléine est : 3*(nombre de moles d'acroléine détectées)/(moles de méthanol entrant + 2*nb de moles d'éthanol entrant).

De même le rendement en acétaldéhyde est : 2*(nombre de moles d'acétaldéhyde détectées)/(moles de méthanol entrant + 2*nb de moles d'éthanol entrant), bien que chimiquement l'acétaldéhyde ait probablement été uniquement obtenu à partir d'éthanol.

**Tableau 1**

| Cata B | Cata A | CH₃OH + CH₃CH₂OH (1/:1 ratio) | O₂ | T | Conversion Méthanol | Conversion Ethanol | Rendement Acroléine | Rendement Acétaldéhyde |
|---|---|---|---|---|---|---|---|---|
| mg | mg | % | % | °C | % | % | % | % |
| Zr 100 | FeMo 100 | 8 | 8 | 325 | 88,5 | 99,5 | 41,1 | 46,3 |
| Zr 100 | FeMo 100 | 8 | 8 | 350 | 91 | 99,2 | 40,9 | 36,8 |
| Zr 67 | FeMo 133 | 8 | 8 | 300 | 87,8 | 99,8 | 47,9 | 30,9 |
| Zr 67 | FeMo 133 | 8 | 8 | 325 | 98,6 | 99,6 | 67,1 | 11,9 |
| Zr 67 | FeMo 133 | 8 | 8 | 350 | 84 | 96 | 7 | 14,7 |
| Zr 133 | FeMo 67 | 8 | 8 | 325 | 88,5 | 99,4 | 43,1 | 42,8 |
| Zr 133 | FeMo 67 | 8 | 8 | 350 | 95,3 | 99,4 | 38,9 | 24,3 |
| Na 100 | FeMo 100 | 8 | 8 | 300 | 79,6 | 99,7 | 33 | 61,1 |
| Na 100 | FeMo 100 | 8 | 8 | 325 | 95,3 | 99,5 | 47,7 | 38 |
| Na 100 | FeMo 100 | 8 | 8 | 350 | 98,6 | 99,8 | 39,9 | 20,3 |
| Na 100 | FeMo 100 | 10 | 10 | 300 | 79,8 | 99,7 | 43,1 | 41,4 |
| Na 100 | FeMo 100 | 10 | 10 | 350 | 98,4 | 99,7 | 47,7 | 13,5 |
| Zr 100 | FeMo 100 | 10 | 10 | 300 | 79,5 | 99,1 | 49,1 | 20,6 |
| Zr 100 | FeMo 100 | 10 | 10 | 350 | 98,4 | 99,6 | 54,4 | 6,7 |
| Zr 67 | FeMo 133 | 8 | 8 | 250 | 38,6 | 83,5 | 6,4 | 43,5 |
| Zr 100 | FeMo 100 | 8 | 8 | 400 | 75,1 | 99 | 18,2 | 40,8 |

### Exemple 4 : synthèse de l'acroléine en présence d'un catalyseur d'oxydation

Dans un réacteur à lit fixe, en verre, de 12 mm de diamètre interne, placé dans un four électrique tubulaire, on charge 200 mg de catalyseur Molybdate de Fer, mélangé avec un volume identique de carbure de silicium de granulométrie 100-125 microns. Le reste du réacteur est ensuite rempli avec du carbure de silicium pour assurer le préchauffage des gaz de réaction, et une bonne distribution des gaz.

Le mélange d'alcool est alimenté au réacteur par une pompe HPLC. Le flux liquide est mélangé au flux gazeux (hélium-oxygène) au niveau d'un évaporateur maintenu à 125 °C pour assurer une vaporisation totale des alcools. Le débit total du flux gazeux envoyé au réacteur est alors de 50 ml/minute, soit une vitesse volumique horaire estimée de 15000 h⁻¹.

Les effluents du réacteur sont refroidis et piégés dans un condenseur, contenant un peu d'eau, et maintenu à 4°C. Les gaz incondensables sont analysés en ligne par chromatographie, et les effluents condensés sont analysés séparément par chromatographie.

La conversion est calculée pour le mélange d'alcools comme étant le nombre de moles de carbone converties sur le nombre de moles de carbone entrantes dans le réacteur. Ceci permet de garder un paramètre unique lorsque l'on fait varier le ratio méthanol/éthanol.

La sélectivité est calculée comme nombre de mole de carbone (dans le produit considéré) sur le nombre de moles de carbone ayant été converties.

On alimente le réacteur avec un mélange méthanol/éthanol de ratio 1,2, avec une pressio partielle totale d'alcools de 7,7 %, et une pression partielle d'oxygène de 7,8 %, pour une pression totale de 1 atmosphère (absolue).

Les produits majoritaires détectés sont l'acroléine et l'acétaldéhyde. La conversion en méthanol et éthanol à 325 °C est totale, et la sélectivité en acroléine est de 48 %. L'acroléine a été produite en présence du seul catalyseur molybdate de fer.

### Exemple 5

On reproduit l'exemple 4 précédent avec un ratio méthanol/éthanol de 0,8, et la même pression partielle totale d'alcools. La sélectivité en acroléine est alors de 57 %, pour une conversion des alcools de 92 %.

### Exemple 6

On reproduit l'exemple 4 précédent avec un ratio méthanol/éthanol de 1,2, et avec une masse de catalyseur 3 fois plus importante (600 mg). La conversion en alcools reste de 100 %, mais la sélectivité en acroléine diminue à 40 % alors que la sélectivité en acétaldéhyde augmente à 22 %.

### Exemple 7

On procède comme à l'exemple 4 précédent, mais en utilisant comme catalyseur d'oxydation, le catalyseur ACF-4 de Nippon Shokubai, qui est un catalyseur de type molybdate de bismuth utilisé notamment pour l'oxydation du propylène en acroléine.

La réaction est effectuée à 300 °C, avec 250 mg de catalyseur. On met en oeuvre un ratio méthanol/éthanol de 1,3, une pression partielle du mélange d'alcools de 8 %, et une pression partielle en oxygène de 10 %.

Le rendement en acroléine est de 18 %.

## Revendications

1. Procédé de synthèse directe d'aldéhydes insaturés de formule CH₂=C(R)-CHO dans laquelle R représente H ou CH₃ à partir d'une charge contenant un mélange de méthanol et d'un second alcool de formule R-CH₂-CH₂OH, consistant à introduire dans un seul ensemble réactionnel comportant un réacteur unique, opéré en phase gazeuse à une température comprise entre 200 et 400 °C et sous une pression comprise entre 1 et 10 bars absolus, contenant un lit catalytique constitué d'un unique catalyseur d'oxydation sélective solide choisi parmi les catalyseurs à base de molybdène ou d'un mélange physique d'un catalyseur d'oxydation sélective solide à base de molybdène avec un catalyseur solide de condensation par aldolisation, une charge contenant le mélange les deux alcools avec de l'oxygène et un gaz diluant non réactif, de telle sorte que le mélange d'alcools et l'oxygène représentent chacun au maximum 10% du volume total du mélange réactionnel puis, à la sortie de l'ensemble réactionnel à recueillir l'effluent gazeux comprenant l'aldéhyde insaturé formé, en présence d'eau coproduite par la réaction.

2. Procédé selon la revendication 1 dans lequel le catalyseur d'oxydation est représenté par la formule générale suivante.
**A**ₐ **X**_{b} **Mo**_{c} **Z**_{d} **O**ₑ (I)
dans laquelle
**A** est au moins un cation choisi parmi les éléments des Groupes 1 à 16 de la Classification Périodique des Eléments et les lanthanides, de préférence un cation d'un métal alcalin tel que Cs, Rb ou K,
**X** est P ou Si, et de préférence P
**Z** est au moins un élément choisi dans le groupe comprenant par W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce and Pb et de préférence Cu, Fe, Bi, Co, Ni, W, V, Cr, Sb, Mn, Ce,
**O** est l'oxygène
a, b, c et d sont des indices constitués de nombres entiers ou décimaux satisfaisant aux plages suivantes
0 ≤ a ≤ 9 et de préférence 0 < a ≤ 9
0 ≤ b ≤ 2 et de préférence 0,1 ≤ b ≤ 1,5
0 < c ≤ 12 et de préférence 5 < c ≤ 12
0 ≤ d ≤ 12 et de préférence 0 < d ≤ 4.
tels que a + b + d > 0
et e est un nombre déterminé par le degré total d'oxydation des éléments.

3. Procédé selon l'une des revendications précédentes dans lequel le catalyseur est un molybdate de fer répondant à la formule générale (I) avec les indices a et b de préférence de valeur 0 et l'indice d de valeur non nulle.

4. Procédé selon l'une des revendications précédentes dans lequel le catalyseur de condensation est choisi parmi les « bases supportées », c'est-à-dire les hydroxydes alcalins LiOH, NaOH, KOH, CsOH déposés sur support silice ou alumine, les métaux alcalins et alcalinoterreux dispersés sur silice, alumine, magnésie, charbon ou carbonate de potassium, les composés azotés, NR₃, NH₃, KNH₂ déposés sur alumine, LiCO₃ déposé sur silice, t-BuOK déposé sur xonotlite.

5. Procédé selon la revendication 4 dans lequel le catalyseur de condensation est choisi parmi le silicate de sodium déposé sur silice ou sur aluminosilicate présentant de préférence un rapport atomique Si/Al supérieur à 10 et comportant le cas échéant un promoteur métallique et le césium déposé sur une silice greffée ou dopée par un composé du zirconium.

6. Procédé selon l'une des revendications 1 à 3 dans lequel le catalyseur de condensation est choisi parmi les « oxydes mixtes » tels que i) SiO₂-MgO, SiO₂-CaO, SiO₂-SrO, SiO₂-BaO, SiO₂-SnO, SiO₂-ZnO, SiO₂-Al₂O₃, SiO₂-ThO₂, SiO₂-TiO₂, SiO₂-ZrO2, SiO₂-MoO₃, SiO₂-WO₃, Al₂O₃-MgO, Al₂O₃-ThO₂, Al₂O₃-TiO₂, Al₂O₃-ZrO₂, Al₂O₃-MoO₃, Al₂O₃-WO₃, ZrO₂-ZnO, ZrO₂-TiO₂, TiO₂-MgO, ZrO₂-SnO₂, ii) les oxydes de type argile comme les hydrotalcites, hydroxyapatites, chrysolite et sépiolite, éventuellement dopés par des alcalins, ainsi que par des métaux tels que le cuivre, le fer, le nickel, iii) les oxydes de terres rares dopés par des alcalino-terreux, iv) les oxydes mixtes de type phosphates mixtes de cobalt et aluminium, v) les silice-alumines dopées avec des sels de Na, K, Cs, Cd, Mg, Ca, Sr, Mn, Zn, Mo, Nb, Pb et/ou Si, vi) MgO-alumine, MgO-SiO₂, vii) les terres rares, sous forme de phosphates, tungstates ou molybdates, viii) les oxynitrures de dérivés phosphorés tels que les oxynitrures mixtes de vanadium-aluminium, phosphore-zirconium, phosphore-aluminium, vanadium-aluminium-phosphore ou gallium-aluminium-phosphore.

7. Procédé selon l'une des revendications précédentes dans lequel on opère à une température comprise entre 250 et 350°C, sous une pression comprise entre 1 et 5 bars absolus.

8. Procédé selon l'une des revendications précédentes dans lequel le débit d'introduction du mélange d'alcools dans l'ensemble réactionnel est tel que la teneur totale en alcools du milieu réactionnel est comprise entre 4 et 10% et de préférence entre 6 et 9%, exprimée en volume.

9. Procédé selon l'une des revendications précédentes dans lequel le mélange d'alcools, méthanol et éthanol ou méthanol et propanol selon les cas, est tel que les ratios molaires méthanol/éthanol et méthanol/propanol sont compris entre 0,8 et 2, de préférence entre 1 et 2.

10. Procédé selon l'une des revendications précédentes dans lequel les réactions sont conduites avec une VVH comprise entre 2 000 et 40 000h⁻¹ et de préférence entre 10 000 et 20 000h⁻¹.

## Patentansprüche

1. Verfahren zur Direktsynthese von ungesättigten Aldehyden der Formel CH₂₌C(R)-CHO, in der R für H oder CH₃ steht, aus einem Einsatzstoff, der eine Mischung von Methanol und einem zweiten Alkohol der Formel R-CH₂-CH₂OH enthält, das darin besteht, dass man in einen einzigen Reaktionsaufbau mit einem einzigen Reaktor, der in der Gasphase eine Temperatur zwischen 200 und 400 °C und unter einem Druck zwischen 1 und 10 bar absolut betrieben wird und eine Katalysatorschüttung enthält, die aus einem einzigen festen selektiven Oxidationskatalysator, der aus Katalysatoren auf Basis von Molybdän ausgewählt ist, oder einer physikalischen Mischung eines festen selektiven Oxidationskatalysators auf Basis von Molybdän mit einem festen Aldolkondensationskatalysator besteht, einen Einsatzstoff, der die Mischung der beiden Alkohole mit Sauerstoff und einem unreaktiven Verdünnungsgas umfasst, derart einträgt, dass die Mischung von Alkoholen und der Sauerstoff jeweils höchstens 10 % des Gesamtvolumens der Reaktionsmischung ausmachen, und dann am Ausgang des Reaktionsaufbaus den gasförmigen Austragsstrom, der den gebildeten ungesättigten Aldehyd in Gegenwart von bei der Reaktion als Nebenprodukt angefallenem Wasser umfasst.

2. Verfahren nach Anspruch 1, bei dem der Oxidationskatalysator durch die folgende allgemeine Formel wiedergegeben wird:
**A**ₐ **X**_{b} **Mo**_{c} **Z**_{d} **O**ₑ (I),
wobei
**A** für mindestens ein Kation, das aus den Elementen der Gruppen 1 bis 16 des Periodensystems der Elemente und den Lanthaniden ausgewählt ist, vorzugsweise ein Kation eines Alkalimetalls wie Cs, Rb oder K, steht,
**X** für P oder Si und vorzugsweise P steht,
**Z** für mindestens ein Element aus der Gruppe umfassend W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce und Pb und vorzugsweise Cu, Fe, Bi, Co, Ni, W, V, Cr, Sb, Mn und Ce ausgewählt ist,
**O** für Sauerstoff steht,
a, b, c und d für Indices stehen, die aus ganzen Zahlen oder Dezimalzahlen bestehen, die den folgenden Bereichen entsprechen:
0 ≤ a ≤ 9 und vorzugsweise 0 < a ≤ 9,
0 ≤ b ≤ 2 und vorzugsweise 0,1 ≤ b ≤ 1,5,
0 < c ≤ 12 und vorzugsweise 5 < c ≤ 12,
0 ≤ d ≤ 12 und vorzugsweise 0 < d ≤ 4,
derart, dass a + b + d > 0,
und e für eine durch die Gesamtoxidationsstufe der Elemente bestimmte Zahl steht.

3. Verfahren nach einem der vorhergehenden Ansprüche, bei dem es sich bei dem Katalysator um ein Eisenmolybdat der allgemeinen Formel (I) handelt, wobei die Indices a und b vorzugsweise den Wert 0 aufweisen und der Index d einen Wert ungleich null aufweist.

4. Verfahren nach einem der vorhergehenden Ansprüche, bei dem der Kondensationskatalysator aus "geträgerten Basen", d. h. auf einem Siliciumdioxid- oder Aluminiumoxid-Träger abgeschiedenen Alkalimetallhydroxiden LiOH, NaOH, KOH, CsOH, auf Siliciumdioxid, Aluminiumoxid, Magnesiumoxid, Kohle oder Kaliumcarbonat dispergierten Alkali- und Erdalkalimetallen, auf Aluminiumoxid abgeschiedenen Stickstoffverbindungen, NR₃, NH₃, KNH₂, auf Siliciumdioxid abgeschiedenem LiCO₃ und auf Xonotlith abgeschiedenem t-BuOK ausgewählt ist.

5. Verfahren nach Anspruch 4, bei dem der Kondensationskatalysator aus auf Siliciumdioxid oder auf Aluminosilikat, das vorzugsweise ein Si/Al-Atomverhältnis von mehr als 10 aufweist, abgeschiedenem Natriumsilikat, das gegebenenfalls einen Metallpromotor und auf einem mit einer Zirconiumverbindung gepfropften oder dotierten Siliciumdioxid abgeschiedenes Caesium umfasst, ausgewählt ist.

6. Verfahren nach einem der Ansprüche 1 bis 3, bei dem der Kondensationskatalysator aus "Mischoxiden" wie i) SiO₂-MgO, SiO₂-CaO, SiO₂-SrO, SiO₂-BaO, SiO₂-SnO, SiO₂-ZnO, SiO₂-Al₂O₃, SiO₂-ThO₂, SiO₂-TiO₂, SiO₂-ZrO₂, SiO₂-MoO₃, SiO₂-WO₃, Al₂O₃-MgO, Al₂O₃-ThO₂, Al₂O₃-TiO₂, Al₂O₃-ZrO₂, Al₂O₃-MoO₃, Al₂O₃-O₃, ZrO₂-ZnO, ZrO₂-TiO₂, TiO₂-MgO, ZrO₂-SnO₂, ii) Oxiden vom Ton-Typ wie Hydrotalciten, Hydroxyapatiten, Chrysolith and Sepiolith, die gegebenenfalls mit Alkalimetallen sowie mit Metallen wie Kupfer, Eisen, Nickel dotiert sind, iii) Seltenerdmetalloxiden, die mit Erdalkalimetallen dotiert sind, iv) Mischoxiden vom Typ gemischter Cobalt- und Aluminiumphosphate, v) Siliciumdioxid-Aluminiumoxiden, die mit Salzen von Na, K, Cs, Cd, Mg, Ca, Sr, Mn, Zn, Mo, Nb, Pb und/oder Si dotiert sind, vi) MgO-Aluminiumoxid, MgO-SiO₂, vii) Seltenerdmetallen in Form von Phosphaten, Wolframaten oder Molybdaten, viii) Oxidnitriden von Phosphorderivaten, wie gemischten Oxidnitriden von Vanadium-Aluminium, Phosphor-Zirconium, Phosphor-Aluminium, Vanadium-Aluminium-Phosphor oder Gallium-Aluminium-Phosphor ausgewählt ist.

7. Verfahren nach einem der vorhergehenden Ansprüche, bei dem man bei einer Temperatur zwischen 250 und 350 °C unter einem Druck zwischen 1 und 5 bar absolut arbeitet.

8. Verfahren nach einem der vorhergehenden Ansprüche, bei dem die Rate des Eintrags der Mischung von Alkoholen in den Reaktionsaufbau so beschaffen ist, dass der Gesamtgehalt an Alkoholen im Reaktionsmedium zwischen 4 und 10 Vol.-% und vorzugsweise zwischen 6 und 9 Vol.-% liegt.

9. Verfahren nach einem der vorhergehenden Ansprüche, bei dem die Mischung von Alkoholen, je nachdem Methanol und Ethanol oder Methanol und Propanol, so beschaffen ist, dass die Methanol/Ethanol- und Methanol/Propanol-Molverhältnisse zwischen 0,8 und 2, vorzugsweise zwischen 1 und 2, liegen.

10. Verfahren nach einem der vorhergehenden Ansprüche, bei dem die Reaktionen mit einer Katalysatorbelastung zwischen 2000 und 40.000 h⁻¹ und vorzugsweise zwischen 10.000 und 20.000 h⁻¹ durchgeführt werden.

## Claims

1. Process for the direct synthesis of unsaturated aldehydes of formula CH₂=C(R)-CHO, in which R represents H or CH₃, from a feedstock containing a mixture of methanol and a second alcohol of formula R-CH₂-CH₂OH, which process consists in introducing, into a single reaction system comprising a single reactor, operated in the gas phase at a temperature of between 200 and 400°C and under a pressure of between 1 and 10 bar absolute and containing a catalytic bed consisting of a single solid selective oxidation catalyst chosen from molybdenum-based catalysts or of a physical mixture of a molybdenum-based solid selective oxidation catalyst with a solid aldol condensation catalyst, a feedstock containing the mixture of the two alcohols with oxygen and an unreactive diluent gas, such that the alcohol mixture and the oxygen each represent at most 10% of the total volume of the reaction mixture, and then, at the outlet of the reaction system, in recovering the gaseous effluent comprising the unsaturated aldehyde formed, in the presence of water coproduced by the reaction.

2. Process according to Claim 1, in which the oxidation catalyst is represented by the following general formula:
**A**ₐ **X**_{b} **Mo**_{c} **Z**_{d} **O**ₑ (I)
in which:
**A** is at least one cation chosen from the elements of Groups 1 to 16 of the Periodic Table of the Elements and the lanthanides, preferably a cation of an alkali metal, such as Cs, Rb or K,
**X** is P or Si, and is preferably P,
**Z** is at least one element chosen from the group consisting of W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce and Pb and is preferably Cu, Fe, Bi, Co, Ni, W, V, Cr, Sb, Mn or Ce,
**O** is oxygen,
a, b, c and d are indices consisting of integers or decimals corresponding to the following ranges: 0 ≤ a ≤ 9 and preferably 0 < a ≤ 9
0 ≤ b ≤ 2 and preferably 0.1 ≤ b ≤ 1.5
0 < c ≤ 12 and preferably 5 < c ≤ 12
0 ≤ d ≤ 12 and preferably 0 < d ≤ 4
such that a + b + d > 0,
and e is a number determined by the total degree of oxidation of the elements.

3. Process according to either of the preceding claims, in which the catalyst is an iron molybdate corresponding to the general formula (I), with the value of the indices a and b preferably being 0 and the value of the index d not being 0.

4. Process according to one of the preceding claims, in which the condensation catalyst is chosen from the "supported bases", that is to say alkali metal hydroxides LiOH, NaOH, KOH, CsOH deposited on a silica or alumina support, alkali metals and alkaline earth metals dispersed on silica, alumina, magnesia, charcoal or potassium carbonate, nitrogenous compounds NR₃, NH₃, KNH₂ deposited on alumina, LiCO₃ deposited on silica, t-BuOK deposited on xonotlite.

5. Process according to Claim 4, in which the condensation catalyst is chosen from sodium silicate deposited on silica or on aluminosilicate preferably having an Si/Al atomic ratio of greater than 10 and comprising, where appropriate, a metal promoter and cesium deposited on silica grafted with or doped by a zirconium compound.

6. Process according to one of Claims 1 to 3, in which the condensation catalyst is chosen from the "mixed oxides", such as i) SiO₂-MgO, SiO₂-CaO, SiO₂-SrO, SiO₂-BaO, SiO₂-SnO, SiO₂-ZnO, SiO₂-Al₂O₃, SiO₂-ThO₂, SiO₂-TiO₂, SiO₂-ZrO₂, SiO₂-MoO₃, SiO₂-WO₃, Al₂O₃MgO, Al₂O₃-ThO₂, Al₂O₃-TiO₂, Al₂O₃-ZrO₂, Al₂O₃-MoO₃, Al₂O₃-WO₃, ZrO₂-ZnO, ZrO₂-TiO₂, TiO₂-MgO, ZrO₂-SnO₂, ii) clay-type oxides, such as hydrotalcites, hydroxyapatites, chrysolite and sepiolite, optionally doped with alkali metals and also with metals such as copper, iron or nickel, iii) rare earth metal oxides doped with alkaline earth metals, iv) mixed oxides of the mixed cobalt and aluminum phosphate type, v) silicas-aluminas doped with salts of Na, K, Cs, Cd, Mg, Ca, Sr, Mn, Zn, Mo, Nb, Pb and/or Si, vi) MgO-alumina, MgO-SiO₂, vii) rare earth metals in the form of phosphates, tungstates or molybdates, viii) oxynitrides of phosphorus derivatives, such as mixed oxynitrides of vanadium-aluminum, phosphorus-zirconium, phosphorus-aluminum, vanadium-aluminum-phosphorus or gallium-aluminum-phosphorus.

7. Process according to one of the preceding claims, in which the process is carried out at a temperature of between 250 and 350°C under a pressure of between 1 and 5 bar absolute.

8. Process according to one of the preceding claims, in which the rate of introduction of the alcohol mixture into the reaction system is such that the total content of alcohols in the reaction medium is between 4 and 10% and preferably between 6 and 9%, expressed by volume.

9. Process according to one of the preceding claims, in which the alcohol mixture - methanol and ethanol or methanol and propanol depending on the case - is such that the methanol/ethanol and methanol/propanol molar ratios are between 0.8 and 2, preferably between 1 and 2.

10. Process according to one of the preceding claims, in which the reactions are carried out with an HSV of between 2000 and 40 000 h⁻¹ and preferably between 10 000 and 20 000 h⁻¹.
